Europäisches Patentamt

(19) European Patent Office

Office européen des brevets

(11) Publication number : **0 109 962 B2**

# NEW EUROPEAN PATENT SPECIFICATION

(45) Date of publication of the new patent specification : **16.12.92 Bulletin 92/51**

(51) Int. Cl.⁵ : **C07C 5/27,** C07C 4/18

(21) Application number : 82110838.8

(22) Date of filing : 23.11.82

(54) Conversion of xylenes containing ethylbenzene.

(43) Date of publication of application :
13.06.84 Bulletin 84/24

(45) Publication of the grant of the patent :
16.07.86 Bulletin 86/29

(45) Mention of the opposition decision :
16.12.92 Bulletin 92/51

(84) Designated Contracting States :
BE DE FR GB IT NL

(56) References cited :
EP-A- 0 021 445
EP-A- 0 042 754
EP-A- 0 057 016
EP-A- 0 062 261
GB-A- 1 254 463
GB-A- 1 275 953
GB-A- 1 298 292
GB-A- 1 323 744
GB-A-13 838 71
US-A- 3 702 886
US-A- 4 139 600

(56) References cited :
US-A- 4 283 584
CHEMICAL ABSTRACTS, vol. 98, no. 8, February 21, 1983, page 105, no. 56006x, Columbus, Ohio, US; & JP-A-57134423

(73) Proprietor : TORAY INDUSTRIES, INC.
2, Nihonbashi-Muromachi 2-chome Chuo-ku
Tokyo 103 (JP)

(72) Inventor : Iwayama, Kazuyoshi
2-1-17 Tsu-Nishi
Kamakura-shi Kanagawa-ken (JP)
Inventor : Ebitani, Atsushi Grand Heights
Kajigaya B-303 2-7-6 Kajigaya Takatsu-ku
Kawasai-shi Kanagawa-ken (JP)
Inventor : Inoue, Takehisa
2-24-11 Noge
Setagaya-ke Tokyo (JP)
Inventor : Kanai, Atsuo
7-3-12 Sakurayama
Zushi-shi Kanagawa-ken (JP)

(74) Representative : Ritter, Stephen David et al
Mathys & Squire 10 Fleet Street
London EC4Y 1AY (GB)

## Description

Background of the invention

The present invention relates to a catalytic conversion process for xylenes containing ethylbenzene.

Among the xylenes, it is para-xylene that is in the greatest industrial demand at present, so the isomerization technique for converting ortho- and meta-xylenes which are in less demand into para-xylene is industrially important.

In general, industrially utilized xylenes are obtained by aromatic extraction and fractional distillation of reaction products from reforming or cracking of naphthas. But all of the crude xylenes thus obtained contain ethylbenzene in addition to ortho-, meta- and para-xylenes. Therefore, ethylbenzene is removed from such crude xylenes by some suitable means, and para-xylene is produced by the combination of separation and isomerisation steps.

Ethylbenzene may be removed by direct separation thereof or by reaction thereof for conversion into a more useful compound. An example of the former is the distillation process wherein, however, it is necessary to perform an ultra-rectification because of a small difference in boiling point between ethylbenzene and xylenes, and this necessity results in increased equipment cost and running expenses. Thus, the distillation process is disadvantageous from the economic point of view. An example of the latter is a method wherein ethylbenzene is converted to xylenes by using a bifunctional catalyst comprising a platinum component and a solid acid component and at the same time there is performed isomerisation of the xylenes. The method of removing ethylbenzene by this reaction is economically advantageous because it requires no special equipment. But a further improvement is desired because of problems involved therein, for example, platinum used in the above-mentioned bifunctional catalyst is a very expensive noble catalyst, and the conversion of ethylbenzene is restricted by a thermodynamic equilibrium relation between ethylbenzene and xylene isomers.

As the method for overcoming the above-mentioned problems, an increasing attention has recently been placed on a conversion process for ethylbenzene into benzene and ethane by using a catalyst comprising a solid acid component and a hydrogenation component. The hydrodealkylation reaction of ethylbenzene into benzene and ethane has features such that the improvement of conversion is relatively easy because the reaction scarcely undergoes thermodynamic restrictions, and that the resulting benzene is easily separable by distillation, because of a large difference in boiling point from xylenes, and has a high added value as a raw material for synthetic fibers and synthetic resins. As a catalyst for allowing such reaction to proceed efficiently, the present inventors have proposed a mordenite catalyst containing rhenium and/or phosphorus or this mordenite catalyst further containing molybdenum, tungsten and/or vanadium, previously in Japanese Laid Open Patent Publication Nos. 9727/1982, 64623/1982 and 134423/1982. But, to make this reaction more efficient, it is desirable to further improve the catalyst activity and selectivity.

In accordance with the present invention, there is provided a process for de-ethylation of ethylbenzene into benzene and simultaneous isomerisation of ortho- and meta-xylenes into paraxylene in feedstocks containing xylenes and ethylbenzene with a catalyst in the presence of hydrogen, characterised in that a catalyst is used which comprises an acid type mordenite together with an acid type zeolite which exhibits the X-ray diffraction pattern described in Table 1 below, said zeolite being present in the range of 1% to 50% by weight based on the total weight of said zeolite and said mordenite.

Table 1

| d (Å) |
|---|
| 11.2 ± 0.2 |
| 10.1 ± 0.2 |
| 3.86 ± 0.08 |
| 3.72 ± 0.08 |
| 3.66 ± 0.08 |

Description of the Preferred Embodiment

As the mordenite used in the present invention, there may be employed any synthetic or natural zeolite having the crystal structure of mordenite. Of course, a mixture of synthetic and natural zeolites may be used. The mordenite can be produced, for example, by the process disclosed in U.S. Patent No.3,436,174 or European Laid Open Patent Publication No.57,016.

As the zeolite used in the present invention, exhibiting the X-ray diffraction pattern shown in Table 1, there may be employed, for example, ZSM-5 disclosed in U.S. Patent No. 3,894,106, ZSM-8 disclosed in British Patent No. 1,334,243, ZSM-11 disclosed in U.S. Patent No. 9,709,979, Zeta 3 disclosed in German Laid Open Patent Publication No. 2,548,695, zeolite prepared without adding an organic substance into reaction mixture, as disclosed in U.S. Patent No. 4,257,885, or zeolite prepared by adding a carboxyl group-containing organic compound into reaction mixture, as disclosed in European Laid Open Patent Publication No. 57,016.

Without being restricted to those just exemplified above, any other zeolites are employable provided they have a structural characteristic exhibiting the X-ray diffraction pattern shown in Table 1. But, zeolites which exhibit the X-ray diffraction pattern shown in Table 2 below are more preferable.

TABLE 2

| d (Å) | d (Å) |
|---|---|
| 11.2±0.2 | 4.27±0.08 |
| 10.1±0.2 | 3.86±0.08 |
| 6.37±0.1 | 3.75±0.08 |
| 6.00±0.1 | 3.72±0.08 |
| 5.71±0.1 | 3.66±0.08 |
| 5.58±0.1 | 3.00±0.05 |
| 4.37±0.08 | 2.00±0.05 |

The $SiO_2/Al_2O_3$ mole ratio of the zeolite is preferably not less than 10, particularly in the range of 20 to 200 and further 30 to 150.

The weight ratio between the zeolite (hereinafter referred to as the zeolite (a)) which exhibits the X-ray diffraction pattern shown in Table 1, and the mordenite, both contained in the catalyst used in the present invention, is in the range of 1% to 50%, preferably 5% to 30%, in terms of percentage by weight of the zeolite (a) based on the total weight of zeolite (a) and mordenite. The catalyst may contain an additional component or components, for example, an inert alumina, other than the two kinds of zeolites described above.

3

The mordenite and the zeolite (a), before their use in the invention, should be treated into acid type. Acid type zeolites, as well known, have hydrogen ions as cations, and they are obtained usually by ion-exchanging at least some of the alkali metal ions and/or alkaline earth metal ions of zeolites which contain those exchangeable cations, with hydrogen ions and/or ammonium cations as hydrogen ion precursors. Generally, the ion-exchange treatment is carried out by using an aqueous solution which contains and acid and/or an ammonium salt. In this case, both inorganic and organic acids are employable, but the use of inorganic acids is more common. Examples of inorganic acids include hydrochloric, nitric, phosphoric and carbonic acids. Of course, there may be used other inorganic acids provided they contain hydrogen ion. Preferred concentrations of acids vary according to the kind of acids used, so it is difficult to define such concentrations absolutely, but care should be exercised to avoid destruction of the mordenite structure.

Examples of ammonium salts which may be used include inorganic ammonium salts such as ammonium nitrate, ammonium chloride, ammonium sulfate, ammonium carbonate and aqueous ammonia, as well as ammonium salts of organic acids such as ammonium formate, ammonium acetate and ammonium citrate, with inorganic ammonium salts being preferred. Ammonium salts are used as a solution of preferably 0.05 to 4 N, more preferably about 0.1 to 2 N.

As the method of such ion-exchange treatment for the zeolites using the acid and/or ammonium salt solution, both batch method and flow method are employable preferably. Where the treatment is made by the batch method, the solid-liquid ratio should be at least a ratio at which the zeolites can fully contact the liquid, preferably about 1 l/kg or more. A treatment time from about 0.1 to 72 hours is sufficient, preferably from about 0.5 to 24 hours, and a treatment temperature below the boiling point is sufficient, but preferably heat is applied to accelerate the ion-exchange rate. Where the flow method is to be followed, there may be adopted the fixed-bed process and the fluidized-bed process, but it is necessary to take steps to avoid fluid channelling or to avoid the ion exchange treatment becoming non-uniform.

The zeolites after subjected to the ion exchange treatment are washed with water, preferably with distilled water, according to either the batch method or the flow method. In this way, hydrogen ions or ammonium ions as hydrogen ion precursors are introduced in the zeolites.

In the cases of the zeolites ZSM-5, ZSM-8 and ZSM-11, since an organic nitrogen-containing cation is used at the time of their production, they can be changed into acid type zeolites by allowing the said organic nitrogen-containing cation to decompose upon calcining for conversion thereof into hydrogen ion, even without application of the ion exchange treatment using the acid and/or ammonium salt solution.

The hydrogen ion content of the catalyst is preferably in the range of 30% to 90%, more preferably 40% to 80%, in terms of gram ion equivalent based on the total exchangeable cations of the zeolite (a) and the mordenite. A too low hydrogen ion content is not desirable because it would cause deterioration of the reaction activity. A too high hydrogen ion content is not desirable, either, because a disproportionation reaction as a side reaction would be accelerated although the reaction activity would become higher. The zeolites thus ion-exchanged with hydrogen ions, namely, the proton type zeolites, if further subjected to heat treatment at a high temperature, become so-called decationized zeolites. But, decationized zeolites and proton type zeolites are not always distinguishable clearly from each other and are often used indiscriminately. The acid type as referred to herein includes decationized type.

If only the zeolites in the catalyst used in the present invention are acid type zeolites, the remaining cations may be various cations. Particularly, alkaline earth metals are preferred because they are effective in improving the reaction selectivity. To let the zeolites contain alkaline earth metal ions, it is necessary to perform an ion exchange for introducing such ions in addition to the ion exchange for introducing hydrogen ions and/or ammonium ions as hydrogen ion precursors. The ion exchange for introducing alkaline earth metal ions is carried out by treating the zeolites with a solution which contains a compound of an alkaline earth metal. Preferred alkaline earth metals are magnesium, calcium, strontium and barium.

Where it is necessary to perform the ion exchange for introducing alkaline earth metal ions, this ion exchange treatment may be conducted separately from the ion exchange treatment for introducing ammonium ions, or both treatments may be carried out at the same time.

According to a particularly preferred ion-exchanging method in the preparation of the catalyst used in the invention, first the ion exchange treatment for introducing alkaline earth metal ions is conducted and then the same treatment for introducing hydrogen ions and/or ammonium ions as hydrogen ion precursors is performed by a liquid recycle type batch process.

In the reaction of the invention using the catalyst thus prepared, ethylbenzene is hydrodealkylated for conversion to benzene and ethane. Therefore, it is preferable that the catalyst used in the invention contain a component having a certain specific hydrogenation activity. Examples of such component include nickel, cobalt, rhenium, molybdenum, tungsten and vanadium. Noble metals such as platinum and palladium are not preferable in the reaction of the invention. If a platinum-supported catalyst is used in the reaction of the invention,

EP 0 109 962 B2

there occurs a hydrogenation reaction at the benzene nuclei of the xylenes because the hydrogenation activity of platinum is too strong, and therefore such catalyst is not desirable. A preferred amount of rhenium to be added ranges from 0.005 to 3%, more preferably 0.02 to 0.5%, by weight as the rhenium element based on the weight of the entire catalyst. A preferred amount of molybdenum, tungsten or vanadium to be added ranges from 0.1 to 10%, more preferably 0.2 to 5%, by weight as the element based on the weight of the entire catalyst. A too small amount is not effective, while a too large amount causes side reaction, and thus both such amounts are not desirable.

As the method for adding such metal to the other catalyst components, there may be adopted, for example, a kneading method, impregnation method and a method of physically mixing powders with each other. For adding the metal element by the kneading method, a compound of the element may be added and kneaded simultaneously with mixing of the mordenite and the zeolite (a) in their granulation. According to the impregnation method, usually, it is possible to support the element by impregnating the zeolites after granulation into a solution containing a compound of the element, followed by draining and drying. In the process of the present invention, the impregnation method is preferred. As the solvent for dissolving the compound of the element, there may be used organic solvents such as alcohols, not to mention water. The amount of the element to be added can be adjusted by suitably selecting the concentration of the compound of the element in the solution.

Examples of compounds of the element which may be used in the invention include, with respect to rhenium, rhenium oxide, perrhenic acid, ammonium perrhenate and rhenium sulfide; with respect to molybdenum, ammonium molybdate, ammonium paramolybdate, ammonium phosphomolybdate, molybdic acid, molybdenum oxide, molybdenum sulfide and molybdates; and with respect to tungsten, ammonium tungstate, ammonium phosphotungstate, tungsten oxide, tungsten sulfide, tungsten carbide and tungstates.

Either a fixed bed or a fluidized bed may be used as a reaction apparatus in the present invention, but the former is preferred because of simpler construction and easier operation. In the fixed bed process, it is preferable from the standpoint of catalyst effectiveness factor that the particle diameter of the catalyst be as small as possible, provided a too small particle diameter is not desirable because it would cause an increased pressure drop. That is, there exists a preferable range of catalyst diameter, which is from 0.05 to 10 mm, more preferably from 0.1 to 2 mm. As the case may be, molding is needed in order for the catalyst to have such a preferred range of particle diameter, for example, compression molding and extrusion. And in order to improve the moldability or impart strength to the catalyst, there may be used a binder, though it goes without saying that the use of binder may be omitted if molding is attainable to a satisfactory extent without the binder. Preferred examples of the binder include natural clays such as kaolin, bentonite, montmorillonite and acid clay as well as synthetic products such as silica sol, alumina sol and alumina gel. The amount of the binder to be added is not more than 70%, preferably not more than 20%, by weight.

The catalyst used in the present invention is prepared basically through the steps of mixing the two zeolite components, molding and treatment into the acid type, the order of which steps may be selected suitably. For example, both zeolite powders may each be treated into the acid type, then mixed and thereafter molded to obtain the catalyst, or both zeolite powders may be molded and treated into the acid type each independently and then mixed to obtain the catalyst.

As described above, the catalyst thus prepared is dried and subsequently calcined before its use. The drying is performed at 50° to 250°C for over 0.1 hour, preferably 0.5 to 48 hours, and the calcination is performed at 300° to 700°C for over 0.1 hour, preferably at 400° to 600°C for 0.5 to 24 hours. By this calcination the ammonium ions introduced in the zeolites by the ion exchange treatment are converted to hydrogen ions, which in turn are converted to the decationized type as the calcination temperature increases, and the catalyst in such a form is also employable effectively.

The catalyst prepared in the manner described above is used under the following reaction conditions. The operating temperature ranges from 300° to 600°C, preferably from 350° to 550°C, and the operating pressure ranges from atmospheric pressure to 100 bar gauge (100 kg/cm$^2$ · G) preferably from atmospheric pressure to 50 bar gauge (50 kg/cm$^2$ · G). The time factor W/F (g-cat · hr/g-mol feedstock, W: catalyst weight, F: mol feed stock per hour) which means the contact time of reaction is in the range of 0.1 to 200, preferably 1 to 100. It is essential that hydrogen is present in the reaction system. If the hydrogen concentration is too low, the dealkylation reaction of ethylbenzene will not proceed to a sufficient extent and a carbonaceous component will be deposited on the catalyst thus resulting in deterioration of the catalyst activity with the lapse of time. A too high concentration of hydrogen is not desirable, either, because, it would cause an increase of hydrocracking reaction. That is, there exists a preferable range of hydrogen concentration, which is 1 to 50, preferably 3 to 30, in terms of mol ratio of hydrogen to feed stock (H$_2$/F).

As the feed stock there is used a mixture of xylenes containing ethylbenzene, wherein the concentration of ethylbenzene is not specially limited. The concentration of para-xylene in the xylene mixture is preferably below the thermodynamic equilibrium concentration, but it is of course possible, as one mode of use in the pres-

5

ent invention, to use as the feed stock a xylene mixture containing para-xylene of the thermodynamic equilibrium concentration with a view to decreasing the concentration of ethylbenzene.

The feed stock may contain other aromatic components, e.g. benzene, toluene, trimethylbenzene, ethyltoluene, diethylbenzene, ethylxylene, provided their concentrations should be in a low range.

The following examples are given to further illustrate the present invention.

Example 1

14.7 g of a solid sodium hydroxide and 10.5 g of tartaric acid were dissolved in 351 g of water, than 5.24 g of a sodium aluminate solution was added to prepare a homogeneous solution. To this mixed solution was then added, slowly with stirring, 66.0 g of silicic acid powder available commercially as white carbon to prepare a homogeneous, slurried, aqueous reaction mixture having the following composition in terms of mol ratios:

| | |
|---|---|
| $SiO_2/Al_2O_3$ | 100 |
| $H_2O/SiO_2$ | 20 |
| $OH^-/SiO_2$ | 0.24 |
| $TA/Al_2O_3$ | 7 (TA: Tartaric acid) |

The mixture was charged into an autoclave having a capacity of 500 ml and the autoclave was closed. Then, heating was made to 160°C with stirring and crystallization was allowed to take place for 72 hours. Then, after cooling, the product was taken out of the autoclave, washed with distilled water and filtered until the pH was almost neutral, and then dried overnight at 110°C. The product thereby obtained was a zeolite having the X-ray diffraction pattern shown in Table 3. Chemical composition of the zeolite upon analysis proved to be 1.3 $Na_2O \cdot Al_2O_3$, 48.7 $SiO_2$ in a dehydrated state.

TABLE 3
X-ray diffraction pattern

| d (Å) | 100I/Io | d (Å) | 100I/Io |
|---|---|---|---|
| 11.18 | 39 | 3.82 | 80 |
| 10.10 | 32 | 3.76 | 41 |
| 9.82 | 13 | 3.73 | 47 |
| 7.49 | 3 | 3.66 | 29 |
| 6.75 | 5 | 3.49 | 5 |
| 6.41 | 10 | 3.45 | 14 |
| 6.04 | 14 | 3.36 | 6 |
| 5.73 | 11 | 3.32 | 14 |
| 5.60 | 12 | 3.06 | 14 |
| 5.40 | 3 | 3.00 | 13 |
| 5.18 | 2 | 2.87 | 4 |
| 5.06 | 5 | 2.74 | 5 |
| 5.01 | 6 | 2.62 | 4 |
| 4.64 | 6 | 2.49 | 8 |
| 4.39 | 8 | 2.02 | 10 |
| 4.29 | 14 | 2.00 | 11 |
| 4.11 | 13 | 1.92 | 3 |
| 4.02 | 7 | 1.88 | 5 |
| 3.86 | 100 | | |

Example 2

7.49 g of a solid sodium hydroxide and 18.8 g of tartaric acid were dissolved in 325 g of water, then 52.4 g of a sodium aluminate solution was added to prepare a homogeneous solution.

To this mixed solution was then added, slowly with stirring, 66.0 g of silicic acid powder to prepare a homogeneous, slurried, aqueous reaction mixture having the following composition in terms of mol ratios:

| | |
|---|---|
| $SiO_2/Al_2O_3$ | 10 |
| $H_2O/SiO_2$ | 20 |
| $OH^-/SiO_2$ | 0.275 |
| $TA/Al_2O_3$ | 1.25 (TA: tartaric acid) |

The mixture was charged into an autoclave having a capacity of 500 ml and the autoclave was closed. Then, heating was made to 160°C with stirring and crystallization was allowed to take place for 48 hours. Then, after cooling, the product was taken out of the autoclave, washed with distilled water and filtered until the pH was almost neutral, and then dried overnight at 110°C. The product thereby obtained as a mordenite type zeolite having the X-ray diffraction pattern shown in Table 4. Chemical composition of the zeolite upon analysis proved to be 0.96 $Na_2O \cdot Al_2O_3$, 9.7 $SiO_2$ dehydrated state.

TABLE 4
X-ray diffraction pattern

| d (Å) | 100I/Io | d (Å) | 100I/Io |
|---|---|---|---|
| 13.86 | 22 | 3.24 | 39 |
| 10.42 | 7 | 3.21 | 47 |
| 9.18 | 55 | 3.17 | 8 |
| 6.64 | 34 | 3.12 | 4 |
| 6.45 | 26 | 2.90 | 27 |
| 6.13 | 10 | 2.72 | 2 |
| 5.85 | 17 | 2.64 | 2 |
| 5.15 | 3 | 2.56 | 8 |
| 5.08 | 3 | 2.52 | 14 |
| 4.55 | 38 | 2.47 | 4 |
| 4.16 | 4 | 2.44 | 4 |
| 4.01 | 77 | 2.28 | 2 |
| 3.86 | 9 | 2.23 | 3 |
| 3.78 | 13 | 2.16 | 2 |
| 3.65 | 3 | 2.04 | 8 |
| 3.55 | 11 | 1.96 | 8 |
| 3.49 | 100 | 1.92 | 3 |
| 3.40 | 63 | 1.88 | 8 |
| 3.30 | 10 | | |

Example 3

2.2 g of aluminum sulfate ($Al_2(SO_4)_3 \cdot 18H_2O$), 11.3 g of sulfuric acid and 15 g of n-propylamine were dissolved in 231 g of water, then 135 g of sodium silicate No. 3 was added slowly with stirring to prepare as homogeneous a gel-like slurry as possible. The slurry was charged into an autoclave having a capacity of 500 ml and the autoclave was closed. Crystallization was allowed to take place at 160°C for 72 hours with stirring. Then, after cooling, the product was washed with distilled water and filtered. The water-washing and filtration were repeated until the washing was almost neutral, followed by drying overnight at about 110°C. The product thereby obtained was zeolite ZSM-5 containing organic ammonium cations with a $SiO_2/Al_2O_3$ mol ratio of 137 and having the X-ray diffraction pattern shown in Table 5.

8

TABLE 5
X-ray diffraction pattern

| d (Å) | 100I/Io | d (Å) | 100I/Io |
|-------|---------|-------|---------|
| 11.07 | 60 | 3.48 | 5 |
| 10.01 | 36 | 3.44 | 12 |
| 9.72 | 12 | 3.35 | 7 |
| 7.45 | 2 | 3.31 | 11 |
| 6.71 | 6 | 3.25 | 4 |
| 6.37 | 11 | 3.14 | 3 |
| 6.00 | 11 | 3.05 | 9 |
| 5.70 | 9 | 2.99 | 11 |
| 5.58 | 9 | 2.94 | 5 |
| 5.36 | 2 | 2.86 | 3 |
| 5.14 | 3 | 2.78 | 2 |
| 5.03 | 4 | 2.73 | 5 |
| 4.98 | 6 | 2.68 | 2 |
| 4.88 | 1 | 2.61 | 4 |
| 4.62 | 7 | 2.51 | 3 |
| 4.36 | 9 | 2.49 | 5 |
| 4.26 | 14 | 2.42 | 3 |
| 4.09 | 5 | 2.39 | 3 |
| 4.01 | 6 | 2.01 | 9 |
| 3.85 | 100 | 1.99 | 9 |
| 3.81 | 66 | 1.95 | 3 |
| 3.74 | 34 | 1.92 | 3 |
| 3.71 | 46 | 1.87 | 3 |
| 3.65 | 25 | | |

Examples 4-7

10 parts by weight (on absolute dry basis) powder of the crystalline aluminosilicate zeolite prepared in Example 1 was mixed with 45 parts by weight (on absolute dry basis) of a commercially available synthetic sodium type mordenite "Zeolon 100-NA" powder manufactured by Norton Company and 45 parts by weight (on absolute dry basis) of alumina powder as a diluent. Then, 15 parts by weight (as $Al_2O_3$) of alumina sol as a binder was added to the powder mixture. After kneading, the kneaded mass was extruded through a screen of 1 mm-φ. After the extrusion, the molded particles were dried overnight at about 110°C, then classified to 12-24 mesh and thereafter calcined in a muffle furnace at 500°C for 2 hours. This molded product upon analysis proved to contain 1.06 meq/g of exchangeable sodium.

100 g (on absolute dry basis) of the molded product was dipped in a solution of 10 g ammonium chloride dissolved in 200 ml of distilled water and ion-exchanged with ammonium ions for about 1 hour with stirring at times in a water bath at about 90°C, then thoroughly washed with distilled water and dried overnight at about 110°C. The so-treated product upon analysis proved to contain 0.703 and 0.297 meq/g of exchangeable ammonium and sodium, respectively. It was then calcined in a muffle furnace at 500°C for 2 hours. The catalyst thus prepared will be referred to hereinafter as catalyst "A".

On the other hand, 20 g (on absolute dry basis) of a likewise treated product after going through the ion exchange with ammonium ions and subsequent drying was impregnated in 40 ml of an aqueous perrhenic acid containing 0.02 g of rhenium as metal and allowed to stand for 30 minutes at room temperature, then allowed to drain and dried overnight at about 110°C. The dried product was then calcined in a muffle furnace at 500°C for 2 hours. The catalyst thus prepared will be referred to hereinafter as catalyst "B".

Using an aqueous ammonium molybdate solution containing 0.20 g of molybdenum as metal in place of the aqueous perrhenic acid solution, a catalyst was prepared in the same way, which will be referred to hereinafter as catalyst "C".

Furthermore, using an aqueous ammonium tungstate solution containing 0.20 g of tungsten as metal in place of the aqueous perrhenic acid solution, a catalyst was prepared in the same way, which will be referred to hereinafter as catalyst "D".

Then, using the catalysts A, B, C and D, feed stocks each comprising ethylbenzene and xylenes were treated under the conditions shown in Table 6. The results are as set out in the same table.

Examples 8-11

7.5 parts by weight (on absolute dry basis) of the zeolite powder prepared in Example 1 and 92.5 parts by weight (on absolute dry basis) of the mordenite type zeolite powder prepared in Example 2 were mixed together, then 15 parts by weight (as $Al_2O_3$) of alumina sol as a binder was added to the powder mixture. After kneading, the kneaded mass was extruded through a screen of 1 mm-$\phi$, then the molded particles were dried overnight at about 110°C and subsequently classified to 12-24 mesh, followed by calcining in a muffle furnace at 500°C for 2 hours. This molded product upon analysis proved to contain 2.07 meq/g of exchangeable sodium.

40 g (on absolute dry basis) of the molded product was dipped in a solution of 4.0 g ammonium chloride dissolved in 80 ml of distilled water and ion-exchanged with ammonium ions for about 1 hour with stirring at times in a water bath at about 90°C, then thoroughly washed with distilled water and dried overnight at about 110°C. The so-treated product upon analysis proved to contain 1.26 and 0.70 meq/g of exchangeable ammonium and sodium, respectively. It was then calcined in a muffle furnace at 500°C for 2 hours. The catalyst thus prepared will be referred to hereinafter as catalyst "E".

On the other hand, 40 g (on absolute dry basis) of the molded product was dipped in 80 ml of an aqueous solution containing 5% by weight of calcium nitrate and ion-exchanged for about 1 hour with stirring at times on a hot water bath at about 90°C. This treatment with the calcium nitrate solution was repeated three times. Thereafter, the so-treated product was washed thoroughly with distilled water, then dipped in 180 ml of an aqueous solution containing 4.0 g of ammonium chloride and treated for about 1 hour with occasional stirring in a water bath at about 90°C, followed by thorough washing with distilled water and subsequent drying overnight at about 110°C. The treated product upon analysis proved to contain 1.00, 0.89 and 0.13 meq/g of exchangeable ammonium, calcium and sodium, respectively. 20 g (on absolute dry basis) of the treated product was impregnated in 40 ml of an aqueous perrhenic acid solution containing 0.02 g of rhenium as metal and allowed to stand for 30 minutes at room temperature, followed by calcining in a muffle furnace at 500°C for 2 hours. The catalyst thus prepared will be hereinafter referred to as catalyst "F".

Furthermore, 40 g (on absolute dry basis) of the molded product was treated using magnesium nitrate in place of calcium nitrate and subsequently treated with the aqueous ammonium chloride solution and the aqueous perrhenic acid in the same way as above to prepare a catalyst, which will be referred to hereinafter as catalyst "G".

Using strontium nitrate in place of calcium nitrate, a catalyst was prepared in the same manner as above which will be referred to hereinafter as catalyst "H".

A conversion reaction of xylenes containing ethylbenzene was carried out using the catalysts E, F, G and H under the conditions shown in Table 6. The results are as set out in the same table.

Examples 12 and 13

40 parts by weight (on absolute dry basis) of the zeolite ZSM-5 powder prepared in Example 3 and 60 parts by weight (on absolute dry basis) of the mordenite type zeolite powder prepared in Example 2 were mixed together, then 15 parts by weight (as $Al_2O_3$) of alumina sol as a binder was added to the powder mixture. After kneading, the kneaded mass was extruded through a screen of 1 mm-ϕ, then the molded particles were dried overnight at about 120°C and thereafter classified to 12-24 mesh, followed by calcining in a muffle furnace at 500°C for 2 hours. 40 g (on absolute dry basis) of this molded product was dipped in a solution of 6.0 g ammonium chloride dissolved in 80 ml of distilled water and ion-exchanged with ammonium ions for about 1 hour with occasional stirring in a water bath at about 90°C, followed by thorough washing with distilled water, drying overnight at about 110°C and subsequent calcining in a muffle furnace at 500°C for 2 hours. The catalyst thus prepared will be referred to hereinafter as catalyst "I".

On the other hand, 40 g (on absolute dry basis) of the molded product was dipped in 80 ml of an aqueous solution containing 5% by weight of magnesium nitrate and ion-exchanged for about 1 hour with occasional stirring in a water bath at about 90°C. This treatment with the aqueous magnesium nitrate solution was repeated five times. Thereafter, the so-treated product was washed thoroughly with distilled water, then dipped in 80 ml of an aqueous solution containing 6.0 g of ammonium chloride and ion-exchanged for about 1 hour with occasional stirring in a water bath at about 90°C, followed by thorough washing with distilled water and subsequent drying overnight at about 110°C. Exchangeable ammonium, magnesium and sodium contents upon analysis proved to be 0.82, 0.45 and 0.10 meq/g, respectively. 20 g (on absolute dry basis) of this treated product was impregnated in 40 ml of an aqueous perrhenic acid solution containing 0.02 g of rhenium as metal and allowed to stand for 30 minutes at room temperature, then allowed to drain and dried overnight at about 110°C, then calcined in a muffle furnace at 500°C for about 2 hours. The catalyst thus prepared will be referred to hereinafter as catalyst "J".

Using the catalysts I and J, a conversion reaction of xylenes was carried out under the conditions shown in Table 7. The results are as set out in the same table.

Example 14

100 g (on absolute dry basis) of the mordenite type zeolite powder prepared in Example 2 was dipped in 500 ml of an aqueous solution containing 5.0% by weight of calcium nitrate and ion-exchanged for about 1 hour with occasional stirring in a water bath at about 90°C, then filtered and again subjected to the ion exchange treatment with the aqueous calcium nitrate solution, which operation was repeated five times, followed by thorough washing with distilled water. 50 g (on absolute dry basis) of the mordenite type zeolite powder prepared in Example 2 thus ion-exchanged with calcium ions was dipped in an aqueous solution containing 5.0 g of ammonium chloride and ion-exchanged with ammonium ions for about 1 hour with occasional stirring in a water bath at about 90°C, followed by thorough washing with distilled water and subsequent drying overnight at about 110°C.

30 g (on absolute dry basis) powder of this mordenite type zeolite thus ion-exchanged with the aqueous ammonium chloride solution was mixed with 20 g (on absolute dry basis) of the ZSM-5 powder prepared in Example 3. To this powder mixture were added 75 g of alumina sol ($Al_2O_3$ content: 10 wt.%) as a binder and 10 ml of an aqueous perrhenic acid solution containing 0.06 g of rhenium as metal, followed by kneading. The kneaded mass was extruded through a screen of 1 mm-ϕ, then the molded particles were dried overnight at about 110°C and then classified to 12-24 mesh, followed by calcining in a muffle furnace at 500°C for 2 hours. The catalyst thus prepared will be referred to hereinafter as catalyst "K".

Using the catalyst K, a conversion reaction of xylenes containing ethylbenzene was carried out under the conditions shown in Table 7. The results are as set out in the same table.

Comparative Examples 1 and 2

60 parts by weight (on absolute dry basis) of the mordenite type zeolite powder prepared in Example 2 and 40 parts by weight (on absolute dry basis) of alumina powder as a diluent were mixed together, then to this powder mixed was added 15 parts by weight (as $Al_2O_3$) of alumina sol as a binder, followed by kneading. The kneaded mass was extruded through a screen of 1 mm-ϕ, then the molded particles were dried overnight at about 110°C and then classified to 12-24 mesh, followed by calcining in a muffle furnace at 500°C for 2 hours. 20 g (on absolute dry basis) of this molded product was dipped in 40 ml of an aqueous solution containing 2.0 g of ammonium chloride and ion-exchanged with ammonium ions for about 1 hour with occasional stirring in a water bath at about 90°C, then washed thoroughly with distilled water and dried overnight at about 110°C, followed by calcining in a muffle furnace at 500°C for 2 hours. The catalyst thus prepared will be referred to hereinafter as catalyst "M".

40 parts by weight (on absolute dry basis) of the ZSM-5 powder prepared in Example 3 and 60 parts by weight (on absolute dry basis) of alumina powder as a diluent were mixed together, then to this powder mixture was added 15 parts by weight (as $Al_2O_3$) of alumina sol as a binder, followed by kneading. The kneaded mass ws extruded through a screen of 1 mm-φ, then the molded particles were dried overnight at about 110°C and then classified to 12-24 mesh, followed by calcining in a muffle furnace at 500°C for 2 hours. This molded product was treated with the aqueous ammonium chloride solution in the same way as above to prepare a catalyst, which catalyst will be referred to hereinafter as catalyst "L".

Using the catalysts L and M, a conversion reaction of xylenes containing ethylbenzene was carried out under the conditions shown in Table 7. The results are as set out in the same table. Reference to Table 7 shows that the catalyst L affords a high ethylbenzene conversion but a low isomerization to para-xylene and that the catalyst M affords only a low ethylbenzene conversion.

## TABLE 6
### Evaluation of various catalysts

| | Item | | Example 4 | Example 5 | Example 6 | Example 7 | Example 8 | Example 9 | Example 10 | Example 11 |
|---|---|---|---|---|---|---|---|---|---|---|
| | Catalyst | | A | B | C | D | E | F | G | H |
| Reaction conditions | Reaction temperature (°C) | | 400 | 400 | 400 | 400 | 400 | 400 | 400 | 400 |
| | Reaction pressure (bar) | | 12 | 12 | 12 | 12 | 12 | 12 | 12 | 12 |
| | $H_2$/F (mol/mol) | | 4 | 4 | 4 | 4 | 4 | 4 | 4 | 4 |
| | W/F (g-cat · hr/g-mol) | | 30 | 30 | 30 | 30 | 30 | 30 | 30 | 30 |
| Reaction products | $C_7$ wt.% | (Feed stock) | 2.12 | 2.64 | 2.93 | 2.79 | 1.56 | 2.17 | 2.06 | 2.34 |
| | BZ | | 5.52 | 6.55 | 6.95 | 6.54 | 4.66 | 5.89 | 5.30 | 5.55 |
| | TOL | | 2.07 | 2.13 | 2.72 | 2.55 | 1.99 | 1.52 | 1.89 | 1.61 |
| | EB | 19.06 | 9.33 | 8.94 | 7.95 | 8.34 | 10.86 | 10.15 | 10.69 | 10.37 |
| | PX | 2.66 | 17.87 | 18.27 | 18.26 | 18.24 | 18.10 | 18.22 | 18.20 | 17.92 |
| | MX | 55.48 | 40.94 | 41.34 | 41.16 | 41.05 | 41.26 | 41.99 | 41.65 | 41.80 |
| | OX | 22.80 | 18.12 | 18.03 | 17.73 | 17.68 | 18.20 | 18.75 | 18.45 | 18.91 |
| | ET | | 0.57 | 0.26 | 0.29 | 0.32 | 0.38 | 0.21 | 0.31 | 0.27 |
| | TMB | | 1.12 | 1.10 | 1.30 | 1.33 | 0.91 | 0.56 | 0.69 | 0.59 |
| | DEB | | 1.72 | 0.41 | 0.41 | 0.76 | 1.66 | 0.32 | 0.42 | 0.33 |
| | EX | | 0.61 | 0.34 | 0.31 | 0.39 | 0.42 | 0.21 | 0.33 | 0.31 |
| EB conversion wt.% | | | 51.0 | 53.1 | 58.3 | 56.2 | 43.0 | 46.7 | 43.9 | 45.6 |
| PX/XY | | | 23.2 | 23.5 | 23.7 | 23.7 | 23.3 | 23.1 | 23.2 | 22.8 |
| XY recovery | | | 95.0 | 95.9 | 95.3 | 95.1 | 95.8 | 97.6 | 96.7 | 97.1 |

EP 0 109 962 B2

13

TABLE 7
Evaluation of various catalysts

| Item | | | Feed stock | Example 12 (I) | Example 13 (J) | Example 14 (K) | Comparative Example 1 (L) | Comparative Example 2 (M) |
|---|---|---|---|---|---|---|---|---|
| | Catalyst | | | I | J | K | L | M |
| Reaction conditions | Reaction temperature (°C) | | | 370 | 370 | 370 | 370 | 370 |
| | Reaction pressure (bar) | | | 12 | 12 | 12 | 12 | 12 |
| | $H_2$/F (mol/mol) | | | 4 | 4 | 4 | 4 | 4 |
| | W/F (g-cat·hr/g-mol) | | | 30 | 30 | 30 | 30 | 30 |
| Reaction products | $C_7$ wt.% | (Feed stock) | | 1.56 | 2.42 | 2.00 | 1.43 | 0.55 |
| | BZ | | | 6.36 | 6.79 | 5.63 | 5.39 | 0.90 |
| | TOL | | | 1.16 | 0.97 | 0.65 | 0.68 | 0.97 |
| | EB | 19.06 | | 7.25 | 7.95 | 9.95 | 9.02 | 17.11 |
| | PX | 2.66 | | 17.66 | 17.63 | 17.64 | 11.37 | 17.46 |
| | MX | 55.48 | | 42.15 | 42.33 | 42.54 | 46.58 | 42.60 |
| | OX | 22.80 | | 19.08 | 19.35 | 19.51 | 21.80 | 19.42 |
| | ET | | | 0.39 | 0.30 | 0.20 | 0.35 | 0.11 |
| | TMB | | | 0.51 | 0.41 | 0.37 | 0.01 | 0.56 |
| | DEB | | | 2.85 | 1.13 | 1.04 | 2.68 | 0.21 |
| | EX | | | 1.03 | 0.71 | 0.46 | 0.70 | 0.11 |
| EB conversion wt.% | | | | 62.0 | 58.3 | 47.8 | 52.7 | 10.2 |
| PX/XY | | | | 22.4 | 22.2 | 22.1 | 14.3 | 22.0 |
| XY recovery | | | | 97.5 | 98.0 | 98.5 | 98.5 | 98.2 |

Note:
$C_7^-$: non-aromatic components of $C_1$—$C_7$
BZ: benzene
TOL: toluene

EB: ethylbenzene
XY: xylenes
PX: para-xylene

MX: meta-xylene
OX: ortho-xylene
ET: ethyltoluene

TMB: trimethylbenzene
DEB: diethylbenzene
EX: ethylxylene.

## Claims

1.  A process for de-ethylation of ethylbenzene into benzene and simultaneous isomerisation of ortho- and meta-xylenes into para-xylene in feedstocks containing xylenes and ethylbenzene with a catalyst in the presence of hydrogen, characterised in that a catalyst is used which comprises an acid type mordenite together with an acid type zeolite which exhibits the X-ray diffraction pattern described in Table 1 said zeolite being present in the range of 1% to 50% by weight based on the total weight of said zeolite and said mordenite.

2.  A process according to Claim 1, characterised in that the hydrogen ion content of said catalyst is in the range of 30% to 90% in terms of gram ion equivalent based on the total exchangeable cations of said zeolite and said mordenite.

3.  A process according to Claim 2, characterised in that said exchangeable cations of said zeolite and said mordenite are mainly hydrogen ions and alkaline earth metal ions.

4.  A process according to any one of Claims 1 to 3, characterised in that said catalyst further contains rhenium, molybdenum, tungsten or vanadium, or a compound thereof.

5.  A process according to Claim 4, characterised in that the content of rhenium or a compound thereof is in the range of 0.005 to 3.0 percent by weight as element based on the total weight of said catalyst.

6.  A process according to Claim 4, characterised in that the content of molybdenum, tungsten or vanadium, or a compound thereof, is in the range of 0.1 to 10 percent by weight as element based on the total weight of said catalyst.

7.  A process according to any one of Claims 1 to 6, characterised in that said catalyst consists mainly of said mordenite and an acid type zeolite which exhibits the X-ray diffraction pattern described in Table 2.

8.  A process according to any one of Claims 1 to 6, characterised in that said zeolite is ZSM-5, ZSM-8, ZSM-11, Zeta-3, a zeolite prepared without adding an organic substance into reaction mixture, or a zeolite prepared by adding a carboxyl group-containing organic compound into reaction mixture.

9.  A process according to any one of Claims 1 to 8, characterised in that the conversion reaction is carried out at a temperature in the range of 300° to 600°C and at a pressure in the range of atmospheric pressure to 100 bar gauge (100kg/cm$^2$.G).

10. A process according to any one of Claims 1 to 9, characterised in that the amount of hydrogen is in the range of 1 to 50 moles per mole of said xylenes containing ethylbenzene.

## Patentansprüche

1.  Verfahren zur De-Ethylierung von Ethylbenzol zu Benzol und gleichzeitige Isomerisierung von Ortho- und Metaxylolen zu Para-Xylol in Beschikkungen, die Xylole und Ethylbenzol enthalten, mit einem Katalysator in Gegenwart von Wasserstoff, dadurch gekennzeichnet, daß ein Katalysator verwendet wird, der einen Mordenit vom Säuretyp zusammen mit einem Zeolith vom Säuretyp umfaßt, welcher das in Tabelle 1 beschriebene Röntgen-Diffraktionsmuster zeigt, wobei der Zeolith in einer Menge im Bereich von 1 bis 50 Gew.-%, bezogen auf das Gesamtgewicht des Zeoliths und des Mordenits, vorhanden ist.

2.  Verfahren nach Anspruch 1, dadurch gekennzeichnet, daß der Wasserstoffionengehalt des Katalysators im Bereich von 30 % bis 90 % auf Grundlage der Ionenequivalents liegt, bezogen auf die gesamten austauschbaren Kationen des Zeoliths und des Mordenits.

3.  Verfahren nach Anspruch 2, dadurch gekennzeichnet, daß die austauschbaren Kationen des Zeoliths und des Mordenits hauptsächlich Wasserstoffionen und Erdalkalimetallionen sind.

4.  Verfahren nach einem der Ansprüche 1 bis 3, dadurch gekennzeichnet, daß der Katalysator weiter Rhenium, Molybdän, Wolfram oder Vanadium oder eine Verbindung davon enthält.

5.  Verfahren nach Anspruch 4, dadurch gekennzeichnet, daß der Gehalt an Rhenium oder einer Verbindung davon im Bereich von 0,005 bis 3,0 Gew.-% als Element, bezogen auf das Gesamtgewicht des Katalysators, liegt.

6.  Verfahren nach Anspruch 4, dadurch gekennzeichnet, daß der Gehalt an Molybdän, Wolfram oder Vanadium oder einer Verbindung davon im Bereich von 0,1 bis 10 Gew.-% als Element, bezogen auf das Gesamtgewicht des Katalysators, liegt.

7.  Verfahren nach einem der Ansprüche 1 bis 6, dadurch gekennzeichnet, daß der Katalysator hauptsächlich aus dem Mordenit und einem Zeolith vom Säuretyp besteht, der das in Tabelle 2 beschriebene Röntgen-Diffraktionsmuster zeigt.

8.  Verfahren nach einem der Ansprüche 1 bis 6, dadurch gekennzeichnet, daß der Zeolith ZSM-5, ZSM-8, ZSM-11, Zeta-3, ein ohne Zusatz einer organischen Substanz zur Reaktionsmischung hergestellter Zeolith oder ein unter Zusatz einer organischen, Carboxylgruppen enthaltenden Verbindung zur Reaktionsmischung hergestellter Zeolith ist.

9.  Verfahren nach einem der Ansprüche 1 bis 8, dadurch gekennzeichnet, daß die Umwandlungsreaktion bei einer Temperatur im Bereich von 300° bis 600°C und bei einem Druck im Bereich von Atmosphärendruck bis 100 bar Überdruck (100 kg/cm$^2$ G) durchgeführt wird.

10. Verfahren nach einem der Ansprüche 1 bis 9, dadurch gekennzeichnet, daß die Wasserstoffmenge im Bereich von 1 bis 50 mol pro Mol der Ethylbenzol enthaltenden Xylole liegt.


## Revendications

1.  Procédé de déséthylation catalytique de l'éthylbenzène en benzène et isomérisation simultanée d'ortho- et méta-xylènes en para-xylène dans des produits contenant des xylènes et de l'éthyl-benzène, en présence d'hydrogène, caractérisé en ce que l'on utilise un catalyseur comprenant une mordénite de type acide ainsi qu'une zéolite de type acide présentant le diagramme de diffraction de rayons X décrit au tableau 1, ladite zéolite étant présente dans la gamme de 1 % à 50 % en poids par rapport au poids total de ladite zéolite et de ladite mordénite.

2.  Procédé selon la revendication 1, caractérisé en ce que la teneur en ions hydrogène dudit catalyseur est comprise dans la gamme de 30% à 90% en termes d'équivalent ion gramme par rapport aux cations totaux pouvant être échangés de ladite zéolite et de ladite mordénite.

3.  Procédé selon la revendication 2, caractérisé en ce que lesdits cations pouvant être échangés de ladite zéolite et de ladite mordénite sont principalement des ions hydrogène et des ions de métaux alcalino-terreux.

4.  Procédé selon l'une quelconque des revendications 1 à 3, caractérisé en ce que ledit catalyseur contient, en outre, du rhénium, du molybdène, du tungstène ou du vanadium ou un de leurs composés.

5.  Procédé selon la revendication 4, caractérisé en ce que la teneur en rhénium ou composé de celui-ci est dans la gamme de 0,005 à 3,0 pourcent,en poids,en tant qu'élément, par rapport au poids total dudit catalyseur.

6.  Procédé selon la revendication 4, caractérisé en ce que la teneur en molybdène, tungstène ou vanadium, ou composé de ceux-ci, est dans la gamme de 0,1 à 10 pourcent, en poids, en tant qu'élément, par rapport au poids total dudit catalyseur.

7.  Procédé selon l'une quelconque des revendications 1 à 6, caractérisé en ce que ledit catalyseur est principalement constitué par ladite mordénite et d'une zéolite de type acide qui présente le diagramme de diffraction de rayons X décrit au tableau 2.

8.  Procédé selon l'une quelconque des revendications 1 à 6, caractérisé en ce que ladite zéolite est la ZSM-5, la ZSM-8, la ZSM-11, la Zéta-3, une zéolite préparée sans adjonction de substance organique dans le mélange réactionnel, ou une zéolite préparée en ajoutant au mélange réactionnel un composé organique contenant un groupe carboxy.

9. Procédé selon l'une quelconque des revendications 1 à 8, caractérisé en ce que l'on effectue une réaction de conversion à une température comprise dans la gamme de 300°C à 600°C et sous une pression comprise dans la gamme s'étendant de la pression atmosphérique à 100 bars (100 kg/cm$^2$.G).

10. Procédé selon l'une des revendications 1 à 9, caractérisé en ce que la quantité d'hydrogène est comprise dans la gamme de 1 à 50 moles par mole dudit éthyl-benzène contenant des xylènes.